# EUROPEAN PATENT APPLICATION

(11) **EP 4 603 837 A2**
(43) Date of publication of application: **20.08.2025**
(21) Application number: 24222996.1
(22) Date of filing: 23.12.2024
(51) Int. Cl.: G01N 33/574, A61K 31/517, A61K 31/5377, A61K 39/395

(54) **METHOD OF ISOLATING AND ANALYZING CIRCULATING TUMOR CELLS**

(30) Priority: 26.12.2023 US 202363614804 P
(71) Applicant: Fullhope Biomedical Co., Ltd., New Taipei City 24159 (TW)
(72) Inventor: LEE, Jan-Mou, 24159 New Taipei City (TW); FANG, Chih-Hao, 24159 New Taipei City (TW); TU, Chia-Chun, 24159 New Taipei City (TW); LIAO, Wan-En, 24159 New Taipei City (TW); HUANG, Li-Yun, 24159 New Taipei City (TW)
(74) Representative: Engelhard, Markus

(57) **Abstract**

This disclosure provides a method for isolating and analyzing circulating tumor cells (CTCs) in a biological sample by using flow cytometry. By using the method, the responsiveness of a subject having lung cancer or gastric cancer to a course of treatment can be predicted in a quick and simple way.

## Description

### CROSS-REFERENCE TO RELATED APPLICATION

This application claims the benefit of U.S. Provisional Application No. 63/614,804, filed on December 26, 2023, which is hereby incorporated by reference herein for all purposes.

### FIELD OF INVENTION

The disclosure relates to the field of individualized medicine. More specifically, liquid biopsy is applied for individualizing and monitoring treatment in patients with tumors and for diagnosis of tumors. The disclosure relates to a method for identifying and characterizing subpopulations (subgroups) of circulating tumor cells (CTCs) in a population of CTCs in a biological sample, preferably in a blood sample of the patient.

### BACKGROUND OF THE INVENTION:

Lung cancer is the most common cause of cancer death in the world and patients with this disease have a 5-year survival rate less than 15%. Non-small cell lung carcinoma (NSCLC), the predominant histological type of lung cancer, accounts for nearly 85% of lung cancer cases. Epidermal growth factor receptor tyrosine kinase inhibitors (EGFR TKIs) have demonstrated significant efficacy in NSCLC patients with EGFR mutations. These inhibitors are associated with minimal side effects and have been shown to improve quality of life, especially in patients carrying the exon 19 deletion (E19del) or exon 21 point mutation (L858R).

Gastric cancer (GC) is one of the most common malignant tumors in the digestive tract. In 2022, global statistics reported approximately 968,784 new cases and around 660,175 deaths caused by GC. Despite recent declines in both incidence and mortality rates, the detection rates and overall prognosis remain unsatisfactory. With the advancement of precision oncology in clinical practice, molecular targeted therapies for GC have gained substantial attention. Among these, therapies targeting human epidermal growth factor receptor 2 (Her2) have shown notable clinical benefits and are now widely used in treatment regimens.

Obtaining molecular information specific to cancer type is an essential in clinical practice for guiding targeted treatment. Typically, such information is derived from solid tissue biopsies, which require invasive procedures to obtain. However, due to advanced disease stages or overall poor health, some patients may have contraindications or be intolerance to those invasive biopsies, highlighting the need for a safer and more convenient alternative. Additionally, for certain cancers, such as GC, biopsy results may lack sufficient accuracy.

Due to the extreme rarity of CTCs, detecting CTCs within a background of abundant leukocytes requires highly sophisticated methodologies. The challenge of CTC detection is further compounded by their cellular heterogeneity and plasticity, making consistent enrichment from biological samples particularly complex. Developing efficient approaches to analyze these cells from minimal sample volumes is crucial for enhancing detection accuracy and increasing their clinical applicability.

### SUMMARY OF THE INVENTION

In response to the urgent need in the field, this disclosure provides a rapid and straightforward method for isolating and analyzing CTCs to facilitate verification of EGFR mutation or Her2 status in these cells. EGFR mutations or Her2 status were assessed by isolating blood-derived epithelial cells from patients with lung cancer, such as NSCLC, or GC, respectively, before initiating treatments with therapies like EGFR TKIs, EGFR targeting agents, or Her2 targeting therapies. The analysis of small groups of mixed CTCs significantly improved the detection rate of EGFR mutations and Her2 status, compared to detection methods that rely on single CTC analysis or traditional immunohistochemistry (IHC).

In one embodiment, the present disclosure provides a method for analyzing circulating tumor cells (CTCs) in a biological sample of an individual with cancer, comprising:
a) isolating white blood cells (WBCs) and CTCs from the biological sample;
b) gating the CTCs based on forward scatter (FSC), CD45, CK-7/8, CK-14/15/16/19, EpCAM, HLA-A.B.C, and Vimentin (VIM); and
c) testing the CTCs from for mutated epithelial growth factor receptor (mEGFR) L858R or Her2;
wherein the steps b) and c) are performed by flow cytometry.

In a preferred embodiment, the biological sample is peripheral blood.

In a preferred embodiment, the biological sample is approximately 1 mL.

The method described herein enables the isolation and analysis of CTCs from a minimal quantity of biological sample, such as 1 mL of peripheral blood.

In a preferred embodiment, the method may further comprise lysing red blood cells in the biological sample before step a).

In a preferred embodiment, the steps b) and c) may be performed sequentially.

In a preferred embodiment, the cancer may be non-small cell lung cancer (NSCLC) or gastric cancer (GC).

In a preferred embodiment, the method may further comprise identifying the cancer as non-small cell lung cancer (NSCLC) if the CTCs express the markers comprising CD45, CK-7/8⁺, CK-14/15/16/19⁻, EpCAM⁺, HLA-A.B.C⁺, and VIM⁺.

In a preferred embodiment, the method may further comprise administrating to the individual an EGFR tyrosine kinase inhibitor if the CTCs is mEGFR L858R⁺.

In a preferred embodiment, the EGFR tyrosine kinase inhibitor may comprise erlotinib, gefitinib, icotininib, afatinib, dacomitinib, osimertinib, rociletinib, olmutinib, lazertinib and zorifertinib.

In a preferred embodiment, the method may further comprise identifying the cancer as gastric cancer (GC) if the CTCs express the markers comprising CD45⁻, CK-7/8⁺, CK-14/15/16/19⁺, EpCAM⁺, and HLA-A.B.C⁺.

In a preferred embodiment, the method may further comprise administrating to the individual at least one treatment selected from the group consisting of: a Her2 tyrosine kinase inhibitors, an anti-Her2 antibody, a bispecific antibody, a Her2-targeting antibody drug conjugates, and Her2-targeted CAR-T cells if the CTCs is Her2⁺.

In a preferred embodiment, the Her2 tyrosine kinase inhibitors may comprise lapatinib; the anti-Her2 antibody may comprise trastuzumab, pertuzumab, tucatinib and margetuximab; the bispecific antibody may comprise zanidatamab; or the Her2-targeting antibody drug conjugates may comprise trastuzumab emtansine and trastuzumab deruxtecan.

In another embodiment, the present disclosure provides a method for treating a cancer in an individual in need thereof, comprising:
a) gating circulating tumor cells (CTCs) based on forward scatter (FSC), CD45, CK-7/8, CK-14/15/16/19, EpCAM, HLA-A.B.C, and VIM in a biological sample derived from the individual;
b) testing the CTCs for mutated epithelial growth factor receptor (mEGFR) L858R or Her2; and
c) administrating to the individual a cancer therapy to the individual, wherein
   i) the cancer therapy is an EGFR tyrosine kinase inhibitor, if the cancer is identified as non-small cell lung cancer (NSCLC) with the CTCs is mEGFR L858R⁺, or
   ii) the cancer therapy comprises at least one treatment selected from the group consisting of: a Her2 tyrosine kinase inhibitors, an anti-Her2 antibody, a bispecific antibody, a Her2-targeting antibody drug conjugates, and Her2-targeted CAR-T cells if the cancer is identified as gastric cancer (GC) with the CTCs is Her2⁺.

Also, the present disclosure provides for an EGFR tyrosine kinase inhibitor, or a Her2 tyrosine kinase inhibitors, an anti-Her2 antibody, a bispecific antibody, a Her2-targeting antibody drug conjugates, or Her2-targeted CAR-T cells, for use in a method of treating a cancer in an individual in need thereof, said method comprising:
a) gating circulating tumor cells (CTCs) based on forward scatter (FSC), CD45, CK-7/8, CK-14/15/16/19, EpCAM, HLA-A.B.C, and VIM in a biological sample derived from the individual;
b) testing the CTCs for mutated epithelial growth factor receptor (mEGFR) L858R or Her2; and
c) administrating to the individual a cancer therapy to the individual, wherein
   i) the cancer therapy is an EGFR tyrosine kinase inhibitor, if the cancer is identified as non-small cell lung cancer (NSCLC) with the CTCs is mEGFR L858R⁺, or
   ii) the cancer therapy comprises at least one treatment selected from the group consisting of: a Her2 tyrosine kinase inhibitors, an anti-Her2 antibody, a bispecific antibody, a Her2-targeting antibody drug conjugates, and Her2-targeted CAR-T cells if the cancer is identified as gastric cancer (GC) with the CTCs is Her2⁺.

In a preferred embodiment, the EGFR tyrosine kinase inhibitor may comprise erlotinib, gefitinib, icotininib, afatinib, dacomitinib, osimertinib, rociletinib, olmutinib, lazertinib and zorifertinib.

In a preferred embodiment, the Her2 tyrosine kinase inhibitors may comprise lapatinib; the anti-Her2 antibody may comprise trastuzumab, pertuzumab, tucatinib and margetuximab; the bispecific antibody may comprise zanidatamab; or the Her2-targeting antibody drug conjugates may comprise trastuzumab emtansine and trastuzumab deruxtecan.

In another embodiment, the present disclosure provides a method for identifying an individual with a cancer likely to respond to treatment with at least one treatment selected from the group consisting of: an EGFR tyrosine kinase inhibitor, a Her2 tyrosine kinase inhibitors, an anti-Her2 antibody, a Her2-targeting antibody drug conjugates, and Her2-targeted CAR-T cells, comprising:
a) gating circulating tumor cells (CTCs) based on forward scatter (FSC), CD45, CK-7/8, CK-14/15/16/19, EpCAM, HLA-A.B.C, and VIM in a biological sample derived from the individual;
b) testing the CTCs for mutated epithelial growth factor receptor (mEGFR) L858R or Her2; and
c) identifying an individual with a cancer likely to respond to treatment with
   i) an EGFR tyrosine kinase inhibitor, if the CTCs are derived from NSCLC and have CD45CK⁻7/8⁺CK-14/15/16/19⁻EpCAM⁺HLA-A.B.C⁺VIM⁺mEGFR L858R⁺; or
   ii) at least one treatment selected from the group consisting of: a Her2 tyrosine kinase inhibitors, an anti-Her2 antibody, a bispecific antibody, a Her2-targeting antibody drug conjugates, and Her2-targeted CAR-T cells, if the CTCs are derived from GC and have CD45⁻CK-7/8⁺CK-14/15/16/19⁺EpCAM⁺HLA-A.B.C⁺Her2⁺.

In another embodiment, the present disclosure provides a kit for use in the method as described herein, comprising one or more reagents for labeling CD45, CK-7/8, CK-14/15/16/19, EpCAM, HLA-A.B.C, VIM, mEGFR L858R and/or Her2.

By examining mixed populations of CTCs, the flow-based method enhances the sensitivity and efficiency of detecting these biomarkers, providing a more robust approach for identifying cancer-related mutations and markers in circulating tumor cells. This improvement highlights the potential of mixed CTC analysis as an alternative for cancer diagnostics and monitoring.

### BRIEF DESCRIPTION OF THE DRAWINGS

The patent application file contains at least one drawing executed in color. Copies of this patent application publication with color drawings will be provided by the USPTO upon request and payment of the necessary fee.

Illustrative embodiments of the present application are described in detail below with reference to the following figures:

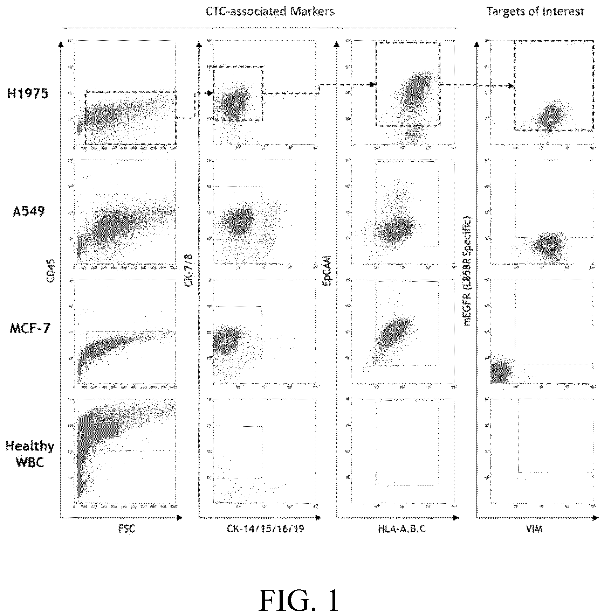

**FIG. 1** illustrates the phenotypic screening of H1975 (NSCLC, mutant EGFR L858R), A549 (NSCLC, wild-type EGFR), and MCF-7 (breast cancer, control) cancer cell lines, as well as healthy donor white blood cells.

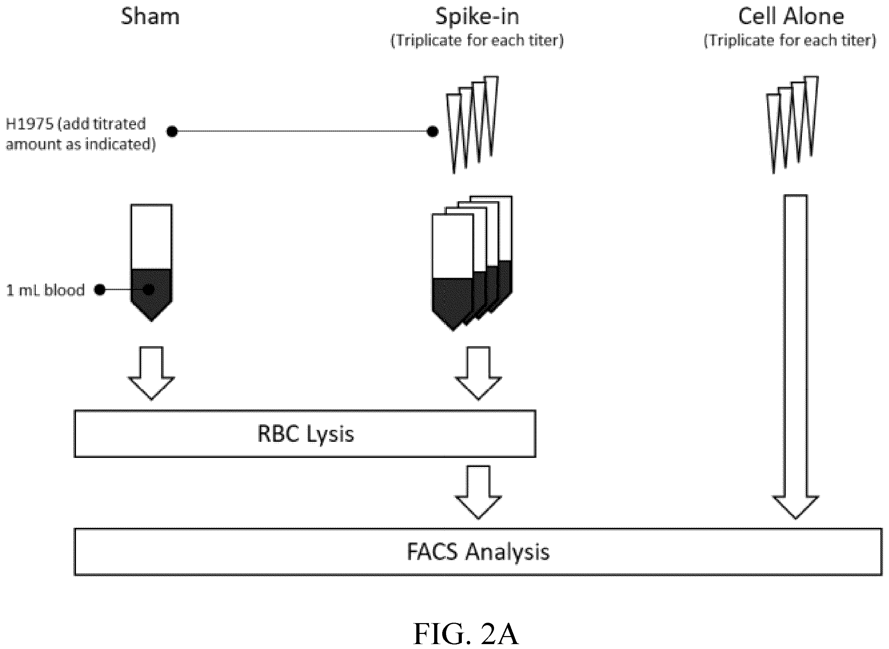

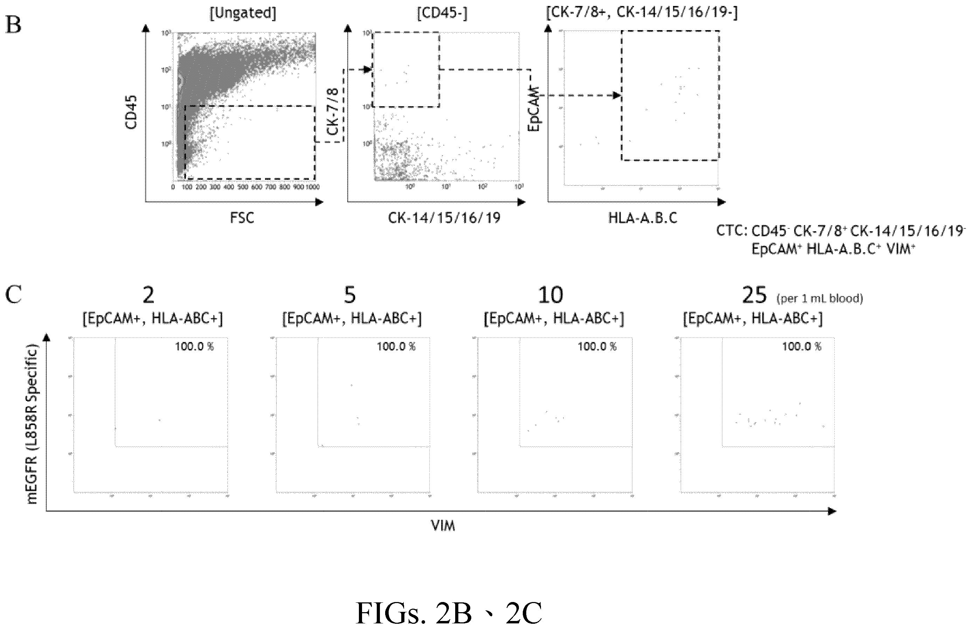

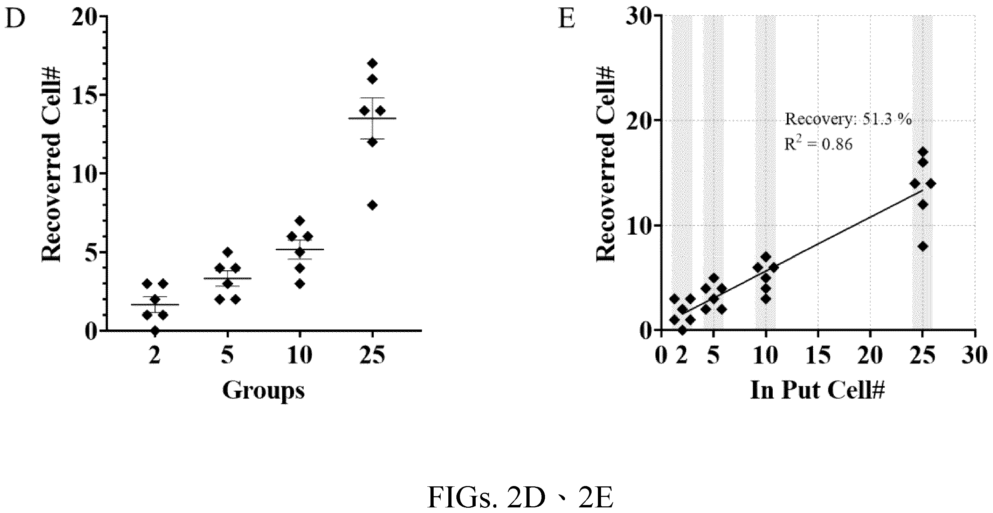

**FIGs. 2**A-D illustrate the recovery validation assay of H1975 cells in a spike assay format. **FIG. 2**A outlines the step-by-step procedure for sample preparation prior to validation. **FIG.2**B shows the selection process of CTC-associated markers applied to each spiked sample. **FIG. 2**C presents a dot plot identifying recovered H1975 cells using specific targeting markers across the spiked groups. Each panel displays representative data from six independent experiments. **FIG. 2**D shows the recovered cell counts of spiked H1975 cells, summarized in a staggered bar graph and presented as MEAN ± SEM. **FIG. 2**E illustrates the systemic recovery rate across all experiments, calculated by linear regression analysis. Each data point represents an independent experiment.

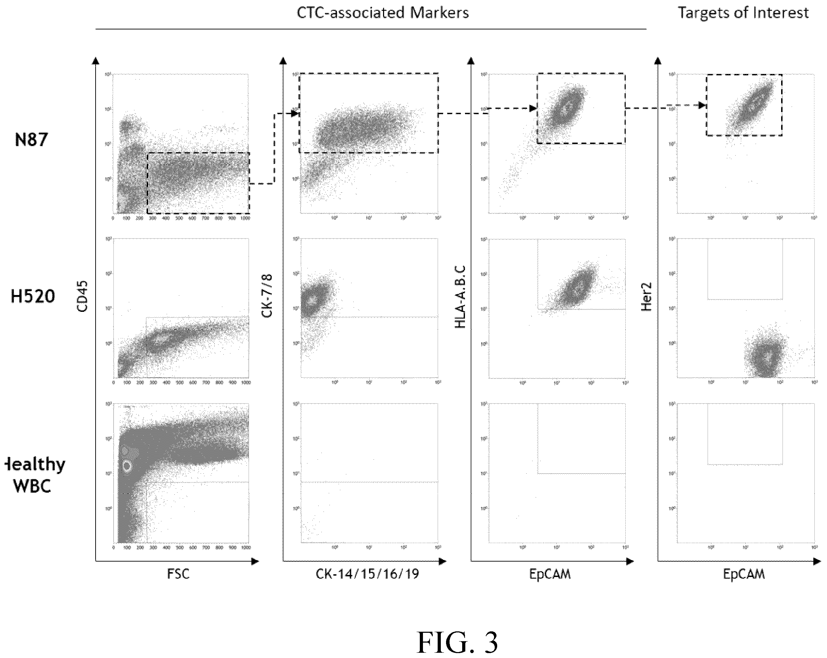

**FIG. 3** illustrates the phenotypic screening of N87 (gastric cancer, Her2⁺), H520 (NSCLC, Her2 control), and healthy donor white blood cells.

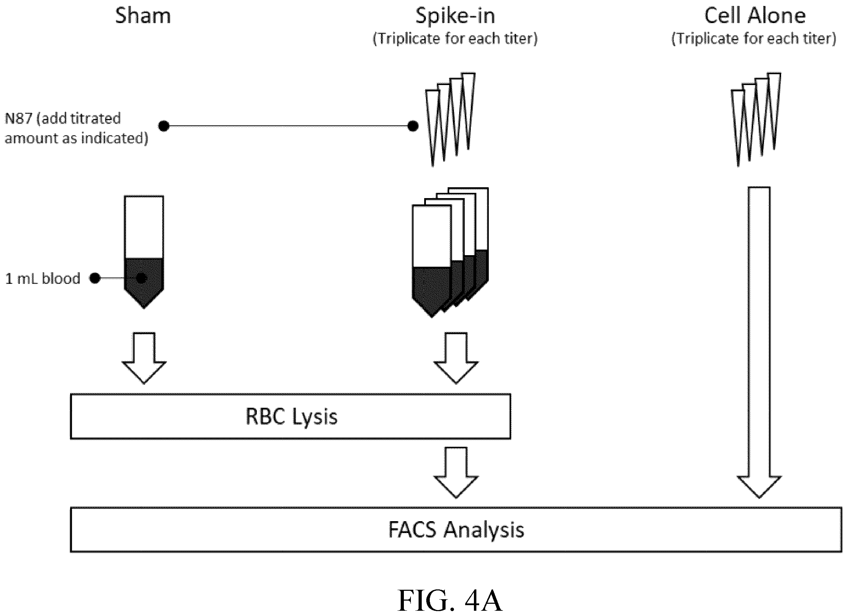

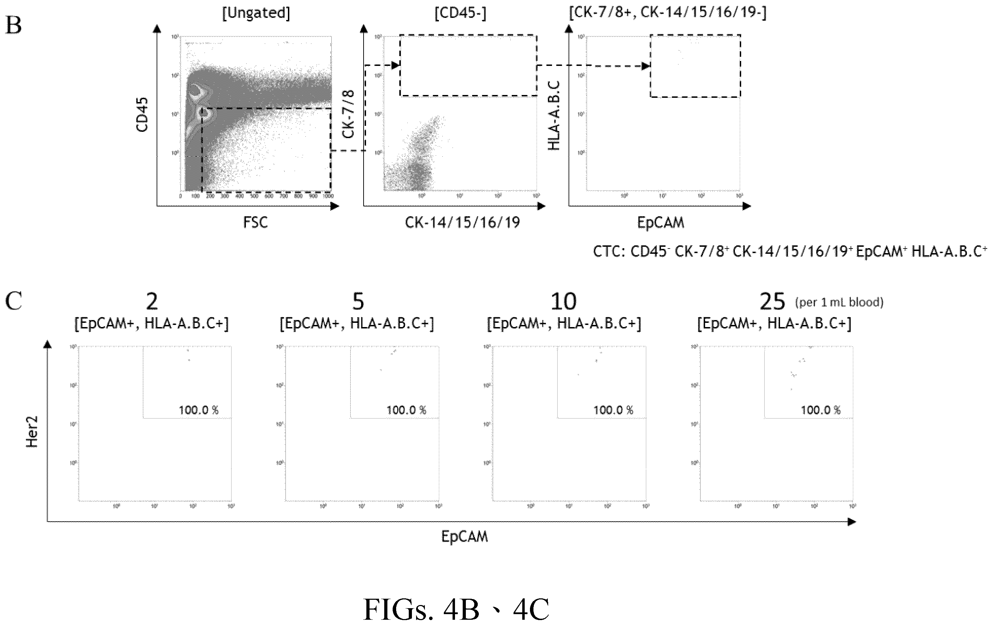

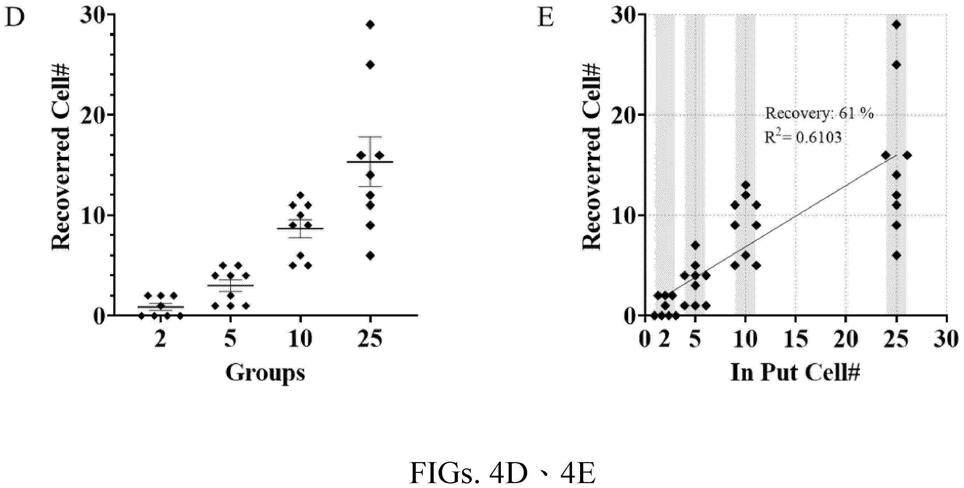

**FIGs. 4**A-D illustrate the recovery validation assay of N87 cells in a spike assay format. **FIG. 4**A outlines the step-by-step procedure for sample preparation prior to validation. **FIG. 4B** shows the selection process of CTC-associated markers applied to each spiked sample. **FIG. 4C** presents a dot plot identifying recovered N87 cells using specific targeting markers across the spiked groups. Each panel displays representative data from at least eight independent experiments. **FIG. 4**D shows the recovered cell counts of spiked N87 cells, summarized in a staggered bar graph and presented as MEAN ± SEM. **FIG. 4**E illustrates the systemic recovery rate across all experiments, calculated by linear regression analysis. Each data point represents an independent experiment.

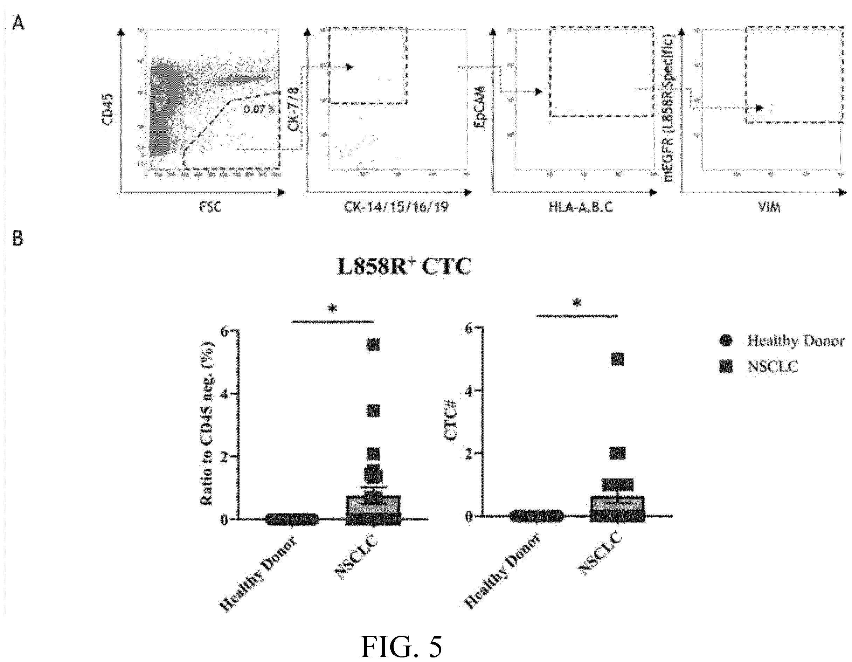

**FIGs. 5**A-B illustrate the validation of a CTC predictive biomarker detection platform for non-small cell lung cancer (NSCLC) using real-world samples. **FIG. 5A** shows that the same pedigree applied in the H1975 recovery assay is used to analyze peripheral blood samples from NSCLC and lung adenocarcinoma patients, as well as healthy donors. **FIG. 5**B presents the validation results, summarizing (1) the percentage of CTCs within the CD45⁻ population and (2) the absolute CTC counts (CTC#). Data are presented as MEAN ± SEM, with each data point representing an individual participant recruited in the IRB-approved study.

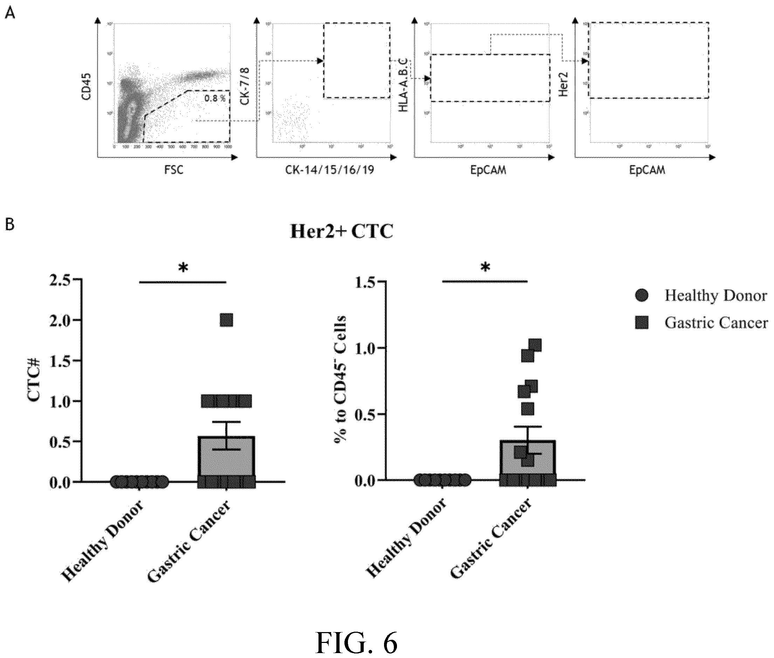

**FIGs. 6**A-B illustrate the validation of a CTC predictive biomarker detection platform for gastric cancer (GC) using real-world samples. **FIG. 6**A shows that the same pedigree used in the GC recovery assay is applied to analyze peripheral blood samples from GC patients and healthy donors. **FIG. 6**B. presents the validation results, summarizing (1) the percentage of CTCs within the CD45⁻ population and (2) the absolute CTC counts (CTC#). Data are presented as MEAN ± SEM, with each data point representing an individual participant recruited in the IRB-approved study.

### DETAILED DESCRIPTION

The foregoing and other aspects of the present disclosure will now be described in more detail with respect to other embodiments described herein. It should be appreciated that the invention can be embodied in different forms and should not be construed as limited to the embodiments set forth herein. Rather, these embodiments are provided so that this disclosure will be thorough and complete, and will fully convey the scope of the invention to those skilled in the art.

The terminology used in the description of the invention herein is for the purpose of describing particular embodiments only and is not intended to be limiting of the invention. As used in the description of the invention and the appended claims, the singular forms "a", "an" and "the" are intended to include the plural forms as well, unless the context clearly indicates otherwise.

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process, or method.

The transitional phrase "consisting of" excludes any elements, steps, or ingredients not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

Where applicants have defined an invention or a portion thereof with an open-ended term such as "comprising," it should be readily understood that (unless otherwise stated) the description should be interpreted to also describe such an invention using the term "consisting of."

All numbers herein may be understood as modified by "about." As used herein, the term "about" is used to indicate that a value includes for example, the inherent variation of error for a measuring device, the method being employed to determine the value, or the variation that exists among the study subjects. Typically, the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability depending on the situation.

The use of the term "or" in the claims is used to mean "and/or" unless explicitly indicated to refer to alternatives only or the alternatives are mutually exclusive, although the disclosure supports a definition that refers to only alternatives and "and/or."

"Subject" as used herein refers to animals, including, for example, a mammalian subject diagnosed with or suspected of having or developing cancer(s). In an embodiment, the term "subject" can refer to a vertebrate having cancer or to a vertebrate deemed to be in need of cancer treatment. Subjects include warm-blooded animals, such as mammals, such as a primate, and, more preferably, a human. Non-human primates are subjects as well. The term subject includes domesticated animals, such as cats, dogs, apes, etc., livestock (for example, cattle, horses, pigs, sheep, goats, etc.) and laboratory animals (for example, mouse, rabbit, rat, gerbil, guinea pig, etc.). Thus, veterinary uses and medical formulations are contemplated herein.

"Administering" or "Administration" is referred to herein as providing a modified T cell or a pharmaceutical composition of the present application to a subject. By way of example and not limitation, administration may be performed via parenteral, subcutaneous, intramuscular, intravenous, intra-articular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, and transdermal. For example, injection may be performed by intravenous (*i.v*.) injection, sub-cutaneous (*s*.*c*.) injection, intradermal (*i.d.*) injection, intraperitoneal (*i.p.*) injection, or intramuscular (*i.m.*) injection. One or more such routes may be employed. Parenteral administration can be, for example, by bolus injection or by gradual perfusion over time. Alternatively, or concurrently, administration may be by the oral route.

The use of the terms "treating," or "treatment" is referred to herein as administration of a treatment or a therapy to a subject with the purpose to cure, alleviate, relieve, remedy, prevent, or ameliorate a disorder, symptoms of the disorder, a disease state secondary to the disorder, or predisposition toward the disorder. The terms "inhibiting," "reducing," or "prevention," or any variation of these terms, when used in the claims and/or the specification includes any measurable decrease or complete inhibition to achieve a desired result.

In certain embodiments, it is desired to limit, reduce, or ameliorate the size of tumor or cancer lesions. The routes of administration will vary, naturally, with the location and nature of the lesion or site to be targeted, and include, e.g., regional, parenteral, intravenous, intramuscular, and/or systemic administration and formulation. Direct injection or injection into the vasculature or the vessels to and from and within an organ or tissue is specifically contemplated for target areas. Local, regional, or systemic administration also may be appropriate.

"Circulating tumor cells", "CTC" and "CTCs" as used interchangeably herein refer to cells that have shed into the vasculature from a primary tumor and circulate in the bloodstream. CTCs are considered seeds for subsequent growth of additional tumors (metastasis) in vital distant organs, triggering a mechanism that is responsible for the vast majority of cancer-related deaths.

"CD45," as used herein, refers to cluster of differentiation 45 (CD45), also known as protein tyrosine phosphatase, receptor type C and leukocyte common antigen, is encoded by the PTPRC gene. CD45 is used to identify leukocytes. An antibody that binds to CD45 may be used to detect CD45.

"Cytokeratin," as used herein, refers to keratin-containing intermediate filaments found in the intracytoplasmic cytoskeleton of epithelial tissue. Cytokeratin-expressing cancer cells lose their cytokeratin expression after undergoing epithelial-mesenchymal transition (EMT), with up to 20% of cells having no detectable cytokeratin. "Cytokeratin-7," "keratin-7," "CK-7" and "sarcolectin" as used interchangeably herein refers to a protein that in humans is encoded by the KRT7 gene. "Cytokeratin-8," "keratin-8" and "CK-8" as used interchangeably herein refers to a protein that in humans is encoded by the KRT8 gene. Cytokeratin 7 and 8 are two closely related type II cytokeratins characteristic of simple epithelia. Cytokeratin 7 is less widespread than cytokeratin 8 and is expressed in sebaceous and sweat glands and some cells of the inner hair root sheath. Cytokeratin 8 is primarily found in the non-squamous epithelia. Cytokeratin 7 is usually present in adenocarcinomas of lung, breast, endometrioid tumors, and transitional cell carcinoma of the bladder. Combination of cytokeratin 7 and 8 is a useful maker for differentiating adenocarcinomas and ductal carcinomas from squamous cell carcinomas. An antibody that binds to cytokeratin 7/8 may be used to detect cytokeratin 7/8.

As used interchangeably herein, "cytokeratin-14," "keratin-14" and "CK-14" refer to a protein encoded by the KRT14 gene in humans. Similarly, "cytokeratin-15," "keratin-15" and "CK-15" refer to a protein encoded by the KRT15 gene, "cytokeratin-16," "keratin-16" and "CK-16" refer to a protein encoded by the KRT16 gene, and "cytokeratin-19," "keratin-19" and "CK-19" refer to a protein encoded by the KRT19 gene. Cytokeratin-14, 15, 16 and 19 are all type I cytokeratins, collectively known as pan-cytokeratin (pan-CK). In cancer research, especially in studies on CTCs, pan-cytokeratin is often used as a biomarker to identify and isolate epithelial-origin tumor cells, which typically overexpress cytokeratins relative to non-epithelial cells.

"EpCAM," as used herein, refers to the epithelial cell adhesion molecule, a type I glycosylated membrane protein expressed at low levels in various human epithelial tissues but overexpressed in most solid cancers. To date, most studies have used EpCAM as a target marker to identify potential CTCs. Its expression has been shown to be inversely related to cancer prognosis in patients.

"HLA-A.B.C", "Major Histocompatibility Complex, Class I, A, B, C" and "HLA Class I A, B, C" as used interchangeably herein refer to as human leukocyte antigens A, B and C, which are encoded by genes located on the short arm of chromosome 6 (6p21.3). The HLA-A.B.C marker is often used in the detection of CTCs as it aids in differentiating CTCs from non-tumor cells in the bloodstream. Specifically, HLA-A.B.C supports the identification of epithelial cells, distinguishes CTCs from immune cells, detects cells undergoing EMT, and provides insights into immune evasion and cancer progression.

"VIM" and "vimentin" as used interchangeably herein refer to a type III intermediate filament (IF) protein that is expressed in mesenchymal cells, where it serves as the main cytoskeletal component. Due to its role in mesenchymal cell structure, vimentin is often used as a marker for mesenchymally-derived cells and for identifying cells undergoing EMT, both in normal development and metastatic progression, including in the detection of CTCs.

The term "sample," "test sample," "specimen," "biological sample," "sample from a subject," or "subject sample" as used herein interchangeably, means a sample or isolate of blood, tissue, urine, serum, plasma, amniotic fluid, cerebrospinal fluid, placental cells or tissue, endothelial cells, leukocytes, or monocytes, can be used directly as obtained from a subject or can be pre-treated, such as by filtration, distillation, extraction, concentration, centrifugation, inactivation of interfering components, addition of reagents, and the like, to modify the character of the sample in some manner as discussed herein or otherwise as is known in the art.

Methods well-known in the art for collecting, handling and processing urine, blood, serum and plasma, and other body fluids, are used in the practice of the present disclosure. The test sample can comprise further moieties in addition to the analyte of interest, such as antibodies, antigens, haptens, hormones, drugs, enzymes, receptors, proteins, peptides, polypeptides, oligonucleotides or polynucleotides. For example, the sample can be a whole blood sample obtained from a subject. It can be necessary or desired that a test sample, particularly whole blood, be treated prior to immunoassay as described herein, e.g., with a pretreatment reagent. Even in cases where pretreatment is not necessary (e.g., most urine samples, a pre-processed archived sample, etc.), pretreatment of the sample is an option that can be performed for mere convenience (e.g., as part of a protocol on a commercial platform). The sample may be used directly as obtained from the subject or following pretreatment to modify a characteristic of the sample. Pretreatment may include extraction, concentration, inactivation of interfering components, and/or the addition of reagents.

Unless otherwise defined, all technical and scientific terms used herein have the same meaning as commonly understood by one of ordinary skill in the art to which this invention belongs. All publications, patent applications, patents and other references cited herein are incorporated by reference in their entireties for the teachings relevant to the sentence and/or paragraph in which the reference is presented.

### Methods of Isolating and Analyzing Circulating Tumor Cells

In one embodiment, the methods of isolating and analyzing circulating tumor cells (CTCs) population in a biological sample of an individual with cancer, comprises the steps of:
a) isolating white blood cells (WBCs) and CTCs from the biological sample;
b) gating the CTCs based on forward scatter (FSC), CD45, CK-7/8, CK-14/15/16/19, EpCAM, HLA-A.B.C, and Vimentin (VIM); and
c) testing the CTCs for mutated epithelial growth factor receptor (mEGFR) L858R or Her2;
wherein the steps b) and c) are performed by flow cytometry.

A biological sample may comprise a population of CTCs. A population of CTCs may comprise on or more subgroups of CTCs. The composition of subgroup(s) of circulating tumor cells (CTCs) is characteristic for a specific tumor, for example a specific solid tumor. CTCs subpopulations and the respective subgroups of CTCs are therefore surrogate markers for cancer, tumors and/or metastasis. The subgroups of CTCs are in addition characteristic for a particular individual, for example a patient. The individual subgroup CTC composition in the biological sample is specific for a particular patient. It can be used to predict and monitor treatment and disease progression or regression respectively. The CTC subgroups and the respective amounts (ratio) in the population of CTCs or the sample that are identified by the method of the disclosure can therefore be used as specific biomarker for the respective tumor, the therapeutic treatment and the individual patient, these biomarkers, e.g. mEGFR L858R or Her2, are for example suitable for individualized medicine.

The biological sample described herein is taken from one individual. The individual can be a human, preferably a patient with cancer. In another embodiment, the individual can be any mammal. In a preferred embodiment, the biological sample to be isolated and analyzed is a human sample, e.g. whole blood.

In an embodiment, WBCs and CTCs may be isolated using any method that effectively removes cells that could interfere with the analysis of the CTCs. For example, the WBCs and CTCs can be isolated through techniques such as centrifugation, red blood cells (RBCs) lysis or Ficoll-Paque. Centrifugation may include density gradient centrifugation, low-speed centrifugation, high-speed centrifugation, or differential centrifugation. In one embodiment, WBCs and CTCs are isolated by lysis of the RBCs. In another embodiment, WBCs and CTCs are isolated by RBCs lysis followed by centrifugation.

In an embodiment, the method comprises the step of gating the WBCs and the CTCs based on forward scatter (FSC) and CD45 so as to exclude cell debris and clumps and to deplete hematopoietic cells to obtain the CTC-like cells. In an embodiment, the hematopoietic cells are depleted using anti-CD45 antibodies. The circulating tumor cell may be confirmed if CD45 expression is negative. Given that CTCs are extraordinarily rare relative to other circulating cells, the isolation of CTCs involves the identification and exclusion of cells expressing the pan-leukocyte marker CD45.

In addition, the method comprises the step of testing the cells for cytokeratin-7/8 (CK-7/8), cytokeratin-14/15/16/19 (CK-14/15/16/19), epithelial cell adhesion molecule (EpCAM), human leukocyte antigen-A.B.C (HLA-A.B.C) and Vimentin (VIM) to identify the CTCs in one biological sample with testing the CTCs for mutated epithelial growth factor receptor (mEGFR) L858R or Her2 to confirm the subset of the CTCs so as to identify the appropriate drug for the treatment.

In an embodiment, the steps b) and c) in the method of the present disclosure are all performed by flow cytometry so as to isolate and analyze the CTCs in one experiment. In another embodiment, the steps b) and c) may be performed sequentially.

In an embodiment, the biological sample may be peripheral blood, preferably about 1 mL, depending on the need of the method of analysis.

In the present invention, the method described herein allows for the isolation and analysis of CTCs from a small quantity of a biological sample, for example, 1 mL of peripheral blood.

In another embodiment, the method may further comprise lysing red blood cells in the biological sample before step a).

The study of the present invention has shown a method configured to detect CTC concentrations at or above 2 CTCs per 1 mL whole blood. In another embodiment, the method is configured to detect CTC concentrations at or above 5 CTCs per 1 mL whole blood. In other words, if there are two or more CTCs in the whole blood, CTCs can be isolated for further analysis on the phenotypes, e.g. mEGFR L858R or Her2. The example's results show that even when there are as few as two CTCs in a 1 mL biological sample, the R² value of the test results still excellent, demonstrating strong sensitivity for low-concentration detection.

In an embodiment, the cancer may be lung cancer, including small-cell lung carcinoma (SCLC) or non-small cell lung cancer (NSCLC).

In a preferred embodiment, the lung cancer is NSCLC with a phenotype of CD45 CK-7/8⁺CK-14/15/16/19⁻EpCAM⁺HLA-AB.C⁺VIM⁺.

In an embodiment, the method may further comprise identifying the cancer as NSCLC if the CTCs express the markers CD45⁻, CK-7/8⁺, CK-14/15/16/19⁻, EpCAM⁺, HLA-A.B.C⁺, and VIM⁺.

The ability to detect and characterize CTCs has the potential to aid in the treatment of cancer patients. The method may further comprise administrating to the individual an EGFR tyrosine kinase inhibitor if the CTCs is identified as mEGFR L858R⁺.

Examples of EGFR tyrosine kinase inhibitor may include erlotinib, gefitinib, icotininib, afatinib, dacomitinib, osimertinib, rociletinib, olmutinib, lazertinib and zorifertinib.

In an embodiment, the cancer may be gastric cancer (GC), e.g. Her2 positive GC.

In a preferred embodiment, the GC has a phenotype of CD45⁻CK-7/8⁺CK-14/15/16/19⁺EpCAM⁺HLA-A.B.C⁺.

In an embodiment, the method may further comprise identifying the cancer as GC if the CTCs express the markers CD45⁻, CK-7/8⁺, CK-14/15/16/19⁺, EpCAM⁺, and HLA-A.B.C⁺.

The individual may be administrated with at least one treatment selected from the group consisting of: a Her2 tyrosine kinase inhibitors, an anti-Her2 antibody, a bispecific antibody, a Her2-targeting antibody drug conjugates, and Her2-targeted CAR-T cells if the CTCs is identified as Her2⁺.

Examples of Her2 tyrosine kinase inhibitors may include lapatinib; examples of anti-Her2 antibody may include trastuzumab, pertuzumab, tucatinib and margetuximab; an example of bispecific antibody may be zanidatamab; and examples of Her2-targeting antibody drug conjugates may include trastuzumab emtansine and trastuzumab deruxtecan.

In another aspect, the present disclosure provides a method for treating a cancer in an individual in need thereof, comprising:
a) gating circulating tumor cells (CTCs) based on forward scatter (FSC), CD45, CK-7/8, CK-14/15/16/19, EpCAM, HLA-A.B.C, and VIM in a biological sample derived from the individual;
b) testing the CTCs for mutated epithelial growth factor receptor (mEGFR) L858R or Her2; and
c) administrating to the individual a cancer therapy to the individual, wherein
   i) the cancer therapy is an EGFR tyrosine kinase inhibitor, if the cancer is identified as non-small cell lung cancer (NSCLC) with the CTCs is mEGFR L858R⁺, or
   ii) the cancer therapy comprises at least one treatment selected from the group consisting of: a Her2 tyrosine kinase inhibitors, an anti-Her2 antibody, a bispecific antibody, a Her2-targeting antibody drug conjugates, and Her2-targeted CAR-T cells if the cancer is identified as gastric cancer (GC) with the CTCs is Her2⁺.

In a preferred aspect, the EGFR tyrosine kinase inhibitor may comprise erlotinib, gefitinib, icotininib, afatinib, dacomitinib, osimertinib, rociletinib, olmutinib, lazertinib and zorifertinib.

In a preferred aspect, the Her2 tyrosine kinase inhibitors may comprise lapatinib; the anti-Her2 antibody may comprise trastuzumab, pertuzumab, tucatinib and margetuximab; the bispecific antibody may comprise zanidatamab; or the Her2-targeting antibody drug conjugates may comprise trastuzumab emtansine and trastuzumab deruxtecan.

In one another aspect, the present disclosure provides a method for identifying an individual with a cancer likely to respond to treatment with at least one treatment selected from the group consisting of: an EGFR tyrosine kinase inhibitor, a Her2 tyrosine kinase inhibitors, an anti-Her2 antibody, a Her2-targeting antibody drug conjugates, and Her2-targeted CAR-T cells, comprising:
a) gating circulating tumor cells (CTCs) based on forward scatter (FSC), CD45, CK-7/8, CK-14/15/16/19, EpCAM, HLA-A.B.C, and VIM in a biological sample derived from the individual;
b) testing the CTCs for mutated epithelial growth factor receptor (mEGFR) L858R or Her2; and
c) identifying an individual with a cancer likely to respond to treatment with
   i) an EGFR tyrosine kinase inhibitor, if the CTCs are derived from NSCLC and have CD45CK⁻7/8⁺CK-14/15/16/19⁻EpCAM⁺HLA-A.B.C⁺VIM⁺mEGFR L858R⁺; or
   ii) at least one treatment selected from the group consisting of: a Her2 tyrosine kinase inhibitors, an anti-Her2 antibody, a bispecific antibody, a Her2-targeting antibody drug conjugates, and Her2-targeted CAR-T cells, if the CTCs are derived from GC and have CD45⁻CK-7/8⁺CK-14/15/16/19⁺EpCAM⁺HLA-A.B.C⁺Her2⁺.

In one aspect, the present disclosure provides a kit for use in the method as described herein, comprising one or more reagents for labeling CD45, CK-7/8, CK-14/15/16/19, EpCAM, HLA-A.B.C, VIM, mEGFR L858R and/or Her2.

The following examples of specific aspects for carrying out the present invention are offered for illustrative purposes only, and are not intended to limit the scope of the present invention in any way.

### EXAMPLES

### Example 1: Phenotypic Screening of Cell Lines H1975 (NSCLC, Mutant EGFR L858R), A549 (NSCLC, Wild-Type EGFR), MCF-7 (Breast Cancer), in Addition to Healthy Donor White Blood Cells

To establish a flow-based CTC detection platform, a series of gating strategies must first be developed and validated. For the mutant EGFR (mEGFR) L858R NSCLC detection platform, cell lines from various cancers were utilized, including H1975 (NSCLC, with mutant EGFR L858R), A549 (NSCLC, wild-type EGFR), and MCF-7 (breast cancer, serving as an indication control). These cell lines were employed to screen for CTC markers and the mutated NSCLC marker, mEGFR L858R. Additionally, white blood cells from a healthy donor, with red blood cells (RBCs) lysed, were used as a healthy control (CTC markers negative control) and subjected to phenotypic screening by flow cytometry.

CTCs are typically identified using biomarkers that are specifically expressed on cancer cells but not on normal hematologic cells. Many cancers, particularly those with epithelial cell phenotypes, express high levels of epithelial cell surface antigens and cytokeratins, which are common markers of epithelial differentiation. As such, epithelial cell adhesion molecule (EpCAM) and cytokeratins are frequently used to identify cancer cells in mixed populations, distinguishing them from other cells such as white blood cells (WBCs).

In Example 1, three cell surface markers, EpCAM, CD45 and HLA-A.B.C, along with two intracellular markers, CK-7/8 and CK-14/15/16/19, were used to distinguish cancer cells from WBCs and other blood cells. Additionally, Vimentin (VIM) was used to differentiate NSCLC from other cancer (breast cancer in this example), while the mutant EGFR L858R marker was used to distinguish between wild-type and L858R EGFR in NSCLC.

To validate the effectiveness of these markers, human tumor cell lines (H1975, A549 for NSCLC, and MCF-7 for breast cancer), along with white blood cells from a healthy donor, were stained with various antibodies: ECD-anti-human CD45, BV510-anti-human EpCAM, FITC-anti-CK-7/8, Alexa Fluor 647-anti-CK-14/15/16/19, Alexa Fluor 700-anti-HLA-A.B.C, Alexa Fluor 405-anti-Vimentin and PE-anti-mEGFR antibodies (EGF Receptor (L858R Mutant Specific) (43B2) Rabbit mAb (PE Conjugate), Cell Signaling Technology Cat#64716).

As shown in **FIG. 1****,** using this approach, H1975, A549, and MCF-7 cells were successfully identified as CTCs. These three cell lines exclusively expressed EpCAM, CK-7/8, and HLA-A.B.C, with most of cells being negative for CD45 and CK-14/15/16/19. In contrast, no CTCs were detected in the healthy donor samples, which served as the negative control. This demonstrates that the method effectively distinguishes CTCs from blood cells using flow cytometry. Furthermore, NSCLC cells could be differentiated from breast cancer cells based on vimentin expression, and the presence of the L858R mutation in EGFR could be specifically identified using the L858R antibody.

In summary, the phenotype of mEGFR L858R NSCLC (H1975 cells) was characterized based on the expression of circulating tumor cell (CTC)-associated markers: CD45⁻, CK-7/8⁺, CK-14/15/16/19⁻, EpCAM⁺, HLA-A.B.C⁺, and VIM+. As well as targeting markers included mutant EGFR (L858R)⁺. A549 cell line, also a type of NSCLC but with wild-type EGFR, was used as a negative control for mutant EGFR (L858R-specific); similarly, MCF-7 cell line, originating from breast cancer, was also utilized as a negative control for mutant EGFR (L858R-specific). While both A549 and MCF-7 cell lines are cancerous and exhibit CTCs markers, they do not express mEGFR L858R, providing a clear contrast for evaluating L858R positivity in the H1975 cells. This comparison further highlights the potential of the flow cytometry-based method for identifying and characterizing L858R positive CTCs in NSCLC.

Flow cytometry screening results for each group are presented in a row-wise manner, consisting of bi-axis panel sets. Dashed squares indicate gating regions of interest for specific cell populations, while dashed arrows represent gating strategies and logics. Solid arrows indicate the intensity or expression levels of specific markers.

### Example 2: Spiking Experiments Using H1975 Cells

To evaluate the ability of flow cytometry to detect rare mEGFR L858R NSCLC cells at low concentrations, a H1975 cell spiking assay was conducted. A small number of H1975 cells were added to healthy whole blood to simulate NSCLC CTCs at low concentrations. The gating strategies from Example 1 for detecting mEGFR L858R NSCLC were applied to analyze the spiked samples, assessing the sensitivity and recovery rate of the method in identifying rare CTCs amidst normal blood cells.

### 2.1. Sample Processing

As shown in **FIG. 2**A, whole blood sample alone and mixtures of whole blood with H1975 aliquots were prepared first.

H1975 cells were harvested at a confluence of 90% by trypsin treatment. After cell counting, aliquots of the cell suspension containing 2, 5, 10 or 25 cells (spike-in groups) were prepared and added to 1 mL of peripheral blood from a healthy donor. A peripheral blood-only sample served as the negative control (sham group), while a sample containing only H1975 cells (cell-alone group) was used as the positive control (indication positive control) for the CTC gating analysis.

The sham group and the spike-in groups were proceeded to red blood cells (RBCs) lysis. Cells from all the groups were stained with ECD-anti-human CD45, BV510-anti-human EpCAM, FITC-anti-CK-7/8, Alexa Fluor 647-anti-CK-14/15/16/19, Alexa Fluor 700-anti-HLA-A.B.C, Alexa Fluor 405-anti-Vimentin and PE-anti-mEGFR antibodies (EGF Receptor (L858R Mutant Specific) (43B2) Rabbit mAb (PE Conjugate), Cell Signaling Technology Cat#64716), or an isotype control, and analyzed using FACS/flow cytometry to identify spiked H1975 cells.

### 2.2. Identification of Spiked H1975 Cells by FACS

The gating strategies were the same as those used for H1975 phenotype screening, as established in Example 1.

Initially, cell debris, clumps, and leukocytes were excluded using forward scatter (FSC) and CD45⁻ (the left panel of **FIG. 2**B). Next, consensus CTC associated markers were identified: CK-7/8⁺ and CK-14/15/16/19⁻ in the middle panel of **FIG. 2**B. Subsequently, additional consensus CTC associated markers were identified: EpCAM⁺ and HLA-A.B.C⁺ in the right panel of **FIG. 2**B. Finally, VIM⁺ was used as additional NSCLC CTC marker, and the target marker mEGFR (L858R-specific)⁺, which is specifically expressed on H1975, was identified, as shown in **FIG. 2**C. The H1975 cells were successfully detected in the spiked samples, with 2 to 25 H1975 cells added to 1 mL of whole blood. The mEGFR (L858R) mutation was observed in 100% of the identified CTCs.

### 2.3. Linear Analysis and Recovery Rate Assessment of Spiked H1975 Cells

The number of detected H1975 cells was compared to the expected cell count to evaluate the recovery of the method. Identification of the recovered H1975 cells was carried out by screening for specific targeting markers: CD45⁻, CK-7/8⁺, CK-14/15/16/19⁻, EpCAM⁺, HLA-A.B.C⁺, VIM and mEGFR (L858R)⁺. The numbers of spiked cells and the detected cell count were plotted on the x-axis and y-axis, respectively **(****FIG. 2**D). A strong linear relationship between the predicted and detected tumor cells was observed, with an R² value of 0.86 **(****FIG. 2**E). These results demonstrate that the method disclosed can detect a single cell in a mixed population of a million cells, which is essential for identifying rare cell types, such as circulating tumor cells (CTCs).

The mean recovery rate was calculated 51.3% **(****FIG. 2**E), which demonstrates the majority of spiked cells were recovered through the enrichment process, and as little as 2 tumor cells in 1 mL of whole blood can be detected. Overall, the method of the present disclosure is an effective way to increase the capacity to detect CTC by flow cytometry.

CTCs exist at extremely low concentrations within a larger population of cells, making their detection challenging. However, by utilizing a flow-based detection platform, the efficiency of identifying target cells is significantly enhanced compared to the current clinical practice, polymerase chain reaction (PCR). The method not only reduces the total number of cells that need to be analyzed, but also as minimizes sample volume required to detect the cells of interest.

### Example 3: Clinical Validation of the CTC Predictive Biomarker Detecting Platform for NSCLC Using Real-World Samples

Above results suggest that the CTC detection platform for NSCLC can identify low number of tumor cell line that spiked in whole blood with a high recovery rate. To further validate the clinical utility of the same flow cytometry protocol used in the H1975 spiked assay, whole blood samples were collected from 26 cancer patients and 10 healthy subjects for CTCs detection **(Table 1).** For cancer patients, all blood samples were collected before the initiation of any anticancer therapy. The performance of the CTC detection platform in distinguishing L858R NSCLC patients from healthy donors was evaluated. Additionally, the feasibility of using the flow-based method to detect the EGFR L858R mutation in CTCs, as opposed to wild-type or other mutations, was also investigated. The results were then compared to the standard diagnostic method to assess the accuracy and reliability of the flow-based method for detecting this specific EGFR mutation in CTCs.

The peripheral blood samples from NSCLC patients, as well as healthy donors, were analyzed using a method consistent with the one used in the H1975 spiked assay. Circulating tumor cells (CTCs) were characterized using the following markers: CD45⁻, CK-7/8⁺, CK-14/15/16/19⁻, HLA-A.B.C, EpCAM⁺, VIM⁺, and mEGFR (L858R)⁺ and were applied to the clinical samples, as shown in **FIG. 5**A. The L858R⁺ CTC detection results are summarized as the percentage of CTCs within the CD45⁻ population and the absolute CTC counts (CTC#), as displayed in **FIG. 5**B. The data show that L858R⁺ CTCs can be detected in the clinical samples from NSCLC patients, while the same marker was not detected in samples from healthy donors. The results are statistically significant, highlighting the potential of this method for identifying L858R⁺ CTCs in NSCLC patients.

The clinical validation results are summarized in **Table 2,** which compares the standard diagnostic procedure (PCR of solid biopsy) with the CTC detection platform (flow cytometry of liquid biopsy, the method presented in this invention). As shown in **Table 2,** the consistency between the PCR and flow-based methods is high, with an agreement of 87.5% (7/8) in identifying positive cases and 82.4% (14/17) in identifying negative cases. These findings demonstrate the reliability of the flow cytometry-based liquid biopsy method, which aligns closely with the established PCR solid biopsy approach while offering potential advantages in terms of sensitivity and non-invasiveness.

**Table 1. Patient Demographics**

| | NSCLC *N= 25* | Normal Subset *N* = 10 | p-value |
|---|---|---|---|
| Male, *N* (%) | 9 (36 %) | 6 (60.0 %) | |
| Female, *N* (%) | 16 (64 %) | 4 (40.0 %) | |
| Median Age, year (range) | 65.5 (86 - 46) | 61.5 (74 - 50) | = 0.07 |
| Stage, *N* (%) | 1 (0.04 %) | | |
| IIIb | 24 (0.96 %) | | |
| IV | | | |
| EGFR mutation, *N* (%) | | | |
| Wildtype | 9 (36%) | | |
| L858R | 8 (32 %) | | |
| Other Mutation | 8 (32 %) | | |

**Table 2. Clinical Validation Correlating Results from the CTC Predictive Biomarker Detecting Platform (Flow Cytometry of Liquid Biopsy) and Standard Diagnostic Procedures (PCR of Solid Biopsy)**

| **Results** | | mEGFR (L858R)⁺ CTC | |
|---|---|---|---|
| | | + | - |
| PCR of mEGFR (L858R) | + | 7 | 1 |
| | - | 3 | 14 |

### Example 4: Phenotypic Screening of Cell Lines N87 (Gastric Cancer, Her2⁺) and H520 (NSCLC, Her2⁻ Control) Alongside Healthy Donor White Blood Cells

To further establish a flow-based CTC detection platform for Her2⁺ gastric cancer (GC), a series of gating strategies must first be developed and validated. Cell lines from various cancers were utilized, including N87 (GC, Her2⁺) and H520 (NSCLC, serving as a Her2 control). These cell lines were employed to screen for CTC markers and the Her2 marker. Additionally, white blood cells from a healthy donor, with red blood cells (RBCs) lysed, were used as a healthy control (CTC markers negative control) and subjected to phenotypic screening by flow cytometry.

CTCs are typically identified using biomarkers that are specifically expressed on cancer cells but not on normal hematologic cells. Many cancers, particularly those with epithelial cell phenotypes, express high levels of epithelial cell surface antigens and cytokeratins, which are common markers of epithelial differentiation. Consequently, epithelial cell adhesion molecule (EpCAM) and cytokeratins are frequently used to identify cancer cells in mixed populations, distinguishing them from other cells such as white blood cells (WBCs).

In Example 4, three cell surface markers, EpCAM, CD45 and HLA-A.B.C, along with two intracellular markers, CK-7/8 and CK-14/15/16/19, were used to distinguish cancer cells from WBCs and other blood cells. Additionally, the Her2 marker was used to detect Her2 positive gastric cancer.

To validate the effectiveness of these markers, human tumor cell lines (N87 for Her2⁺ GC and H520 for Her2 NSCLC), along with white blood cells from a healthy donor, were stained with various antibodies: ECD-anti-human CD45, BV510-anti-human EpCAM, FITC-anti-CK-7/8, Alexa Fluor 647-anti-CK-14/15/16/19, Alexa Fluor 700-anti-HLA-A.B.C and PE/Cy7-anti-Her2 antibodies.

As shown in **FIG. 3****,** this approach successfully identified N87 and H520 cells as CTCs. Both cell lines exclusively expressed EpCAM, CK-7/8, CK-14/15/16/19 and HLA-A.B.C, with the majority of cells being negative for CD45. In contrast, no CTCs were detected in the healthy donor samples, which served as the negative control. This finding demonstrates that the method effectively distinguishes CTCs from other blood cells using flow cytometry. Furthermore, N87 and H520 cells could be differentiated from one another based on the expression of Her2, highlighting the method's ability to discriminate between CTC populations.

In summary, the phenotype of Her2 positive GC cells (N87 cells) was characterized based on the expression of circulating tumor cell (CTC)-associated markers, including CD45⁻, CK-7/8⁺, CK-14/15/16/19⁺, EpCAM⁺, and HLA-A.B.C⁺, as well as the targeting marker Her2⁺. While H520 cells are cancerous and exhibit CTC markers, they do not express Her2, providing a clear contrast for evaluating Her2 positivity in the N87 cells. This comparison further highlights the potential of the flow cytometry-based method for identifying and characterizing Her2 positive CTCs in GC.

Flow cytometry screening results for each group are presented in a row-wise manner, consisting of bi-axis panel sets. Dashed squares indicate gating regions of interest for specific cell populations, while dashed arrows represent gating strategies and logics. Solid arrows indicate the intensity or expression levels of specific markers.

### Example 5: Spiking Experiments Using N87 Cells

To evaluate the ability of flow cytometry to detect Her2 positive gastric cancer cells at low concentrations, a N87 cell spiking assay was conducted. A small number of N87 cells were added to healthy whole blood to simulate GC CTCs at low concentrations. The gating strategies from Example 4 for detecting Her2 positive GC were applied to analyze the spiked samples, assessing the sensitivity and recovery rate of the method in identifying rare CTCs amidst normal blood cells.

### 5.1. Sample Processing

As shown in **FIG. 4**A, whole blood sample alone and mixtures of whole blood with N87 aliquots were prepared first.

N87 cells were harvested at a confluence of 90% by trypsin treatment. After cell counting, aliquots of the cell suspension containing 2, 5, 10 or 25 cells (spike-in groups) were prepared and added to 1 mL of peripheral blood from a healthy donor. A peripheral blood-only sample served as the negative control (sham group), while a sample containing only N87 cells (cell-alone group) was used as the positive control (indication positive control) for the CTC gating analysis.

The sham group and the spike-in groups were proceeded to red blood cells (RBCs) lysis. Cells from all the groups were stained with ECD-anti-human CD45, BV510-anti-human EpCAM, FITC-anti-CK-7/8, Alexa Fluor 647-anti-CK-14/15/16/19, Alexa Fluor 700-anti-HLA-A.B.C and PE/Cy7-anti-Her2 antibodies, and analyzed using FACS/flow cytometry to identify spiked N87 cells.

### 5.2. Isolation and Analysis of Spiked N87 Cells by FACS

The gating strategies were the same as those used for N87 phenotype screening, as established in Example 4.

Initially, cell debris, clumps, and leukocytes were excluded using forward scatter (FSC) and CD45⁻ (the left panel of **FIG.** 4B). Next, consensus CTC associated markers were identified: CK-7/8⁺ and CK-14/15/16/19⁺ in the middle panel of **FIG. 4**B. Subsequently, additional consensus CTC associated markers were identified: EpCAM⁺ and HLA-A.B.C⁺ in the right panel of **FIG. 4**B. Finally, the target marker Her2⁺, which is specifically expressed on N87, was identified, as shown in **FIG. 4**C. The N87 cells were successfully detected in the spiked samples, with 2 to 25 N87 cells added to 1 mL of whole blood. The Her2⁺ cells was observed in 100% of the identified CTCs.

### 5.3. Linear Analysis and Recovery Rate Assessment of Spiked N87 Cells

The number of detected N87 cells was compared to the expected cell count to evaluate the recovery of the method. Identification of the recovered N87 cells was carried out by screening for specific targeting markers: CD45⁻, CK-7/8⁺, CK-14/15/16/19⁺, EpCAM⁺, HLA-A.B.C⁺, and Her2⁺. The numbers of spiked cells and the detected cell count were plotted on the x-axis and y-axis, respectively **(****FIG. 4**D). A strong linear relationship between the predicted and detected tumor cells was observed, with an R² value of 0.6103 **(****FIG. 4**E). These results demonstrate that the method disclosed can detect a single cell in a mixed population of a million cells, which is essential for identifying rare cell types, such as circulating tumor cells (CTCs).

The mean recovery rate was calculated 61% **(****FIG. 4**E), which demonstrates the majority of spiked cells were recovered through the enrichment process, and as little as 2 tumor cells in 1 mL of whole blood can be detected. Overall, the method of the present disclosure is an effective way to increase the capacity to detect CTC by flow cytometry.

CTCs exist at extremely low concentrations within a larger population of cells, making their detection challenging. However, by utilizing a flow-based detection platform, the efficiency of identifying Her2 positive GC is significantly enhanced compared to the current clinical practice, immunohistochemistry (IHC). This approach not only reduces the total number of cells that need to be analyzed, but also as minimizes sample volume required to detect the cells of interest.

### Example 6: Validation of a CTC Predictive Biomarker Detection Platform for GC Using Real-World Samples

The results above suggest that the CTC detection platform for GC is capable of identifying low number of tumor cell line that spiked in whole blood with a high recovery rate. To validate the clinical utility of the same flow cytometry protocol used in the N87 spiked assay, whole blood samples were collected from 14 cancer patients and 10 healthy subjects for CTCs detection **(Table 3).** All blood samples from cancer patients were collected before starting anticancer therapy. The performance of the CTC detection platform in distinguishing Her2⁺ GC patients from healthy donors was evaluated. Additionally, the correlation between the standard diagnosis method and the CTC detection method was also assessed.

The peripheral blood samples from gastric cancer (GC) patients, as well as healthy donors, were analyzed using a method consistent with the one used in the GC recovery assay. Circulating tumor cells (CTCs) were characterized using the following markers: CD45⁻, CK-7/8⁺, CK-14/15/16/19⁺, EpCAM⁺, HLA-A.B.C⁺, and Her2⁺. These markers were applied to the clinical samples, as shown in **FIG. 6**A. The Her2⁺ CTC detection results are summarized as the percentage of CTCs within the CD45⁻ population and the absolute CTC counts (CTC#), as displayed in **FIG. 6B****.** The data show that CTCs can be detected in the clinical samples from GC patients, while the same marker was not detected in samples from healthy donors. The results are statistically significant, highlighting the potential of this method for identifying Her2⁺ CTCs in GC patients.

The clinical validation results are summarized in **Table 4,** which compares the standard diagnostic procedure (IHC of solid biopsy) with the CTC detection platform (flow cytometry of liquid biopsy, the method presented in this invention). As shown in **Table 4,** the agreement between IHC and the flow-based method is perfect in identifying positive cases, with 7 out of 7 positive cases detected by both methods (100% agreement). Furthermore, both methods agreed on all 7 negative cases (100% agreement), with no false positives or false negatives.

These results demonstrate a perfect correlation between the IHC-based solid biopsy and the flow cytometry-based liquid biopsy methods, showcasing the high accuracy and reliability of the flow cytometry method. The flow-based detection method not only matches IHC in both positive and negative case identification but also offers the advantage of being non-invasive, making it a promising alternative for clinical diagnostics of Her2⁺ CTCs in GC patients.

**Table 3. Patient Demographics**

| | GC *N= 14* | Normal Subset *N= 10* | p-value |
|---|---|---|---|
| Male, *N* (%) | 7 (50 %) | 6 (60.0 %) | |
| Female, *N* (%) | 7 (50 %) | 4 (40.0 %) | = 0.051 |
| Median Age, year (range) | 70 (79 - 50) | 61.5 (74 - 50) | |
| Stage, *N* (%) | | | |
| I | 2 (14.3 %) | | |
| IIa | 2 (14.3 %) | | |
| IIb | 1 (7.1 %) | | |
| IIIa | 2 (14.3 %) | | |
| IIIb | 2 (14.3 %) | | |
| IIIc | 1 (7.1 %) | | |
| IV | 4 (28.6 %) | | |
| Her2⁺, *N* (%) | | | |
| Negative (= 0) | 7 (50 %) | | |
| Positive (> 0) | 7 (50 %) | | |

**Table 4. Clinical Validation Correlating Results from the CTC Predictive Biomarkers Detecting Platform (Flow Cytometry of Liquid Biopsy) and Standard Diagnostic Procedures (IHC of Solid Biopsy)**

| **Results** | | Her2⁺ CTC | |
|---|---|---|---|
| | | + | - |
| IHC of Her2 | + | 7 | 0 |
| | - | 0 | 7 |

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

It should also be understood that, unless clearly indicated to the contrary, in any methods claimed herein that include more than one step or act, the order of the steps or acts of the method is not necessarily limited to the order in which the steps or acts of the method are recited.

## Claims

1. A method for analyzing circulating tumor cells (CTCs) in a biological sample of an individual with cancer, comprising:
a) isolating white blood cells (WBCs) and CTCs from the biological sample;
b) gating the CTCs based on forward scatter (FSC), CD45, CK-7/8, CK-14/15/16/19, EpCAM, HLA-A.B.C, and Vimentin (VIM); and
c) testing the CTCs for mutated epithelial growth factor receptor (mEGFR) L858R or Her2;
wherein steps b) and c) are performed by flow cytometry.

2. The method of claim 1, wherein the biological sample is peripheral blood.

3. The method of claim 2, wherein the biological sample is about 1 mL.

4. The method of claim 1, wherein the biological sample comprises more than 5 CTCs per mL of the biological sample.

5. The method of claim 1, further comprising lysing red blood cells in the biological sample before step a).

6. The method of claim 1, wherein steps b) and c) are performed sequentially.

7. The method of claim 1, further comprising identifying the cancer as non-small cell lung cancer (NSCLC) if the CTCs express the markers comprising CD45⁻, CK-7/8⁺, CK-14/15/16/19⁻, EpCAM⁺, HLA-A.B.C⁺, and VIM⁺.

8. The method of claim 7, further comprising administrating to the individual an EGFR tyrosine kinase inhibitor if the CTCs is mEGFR L858R⁺.

9. The method of claim 8, wherein the EGFR tyrosine kinase inhibitor comprises erlotinib, gefitinib, icotininib, afatinib, dacomitinib, osimertinib, rociletinib, olmutinib, lazertinib and zorifertinib.

10. The method of claim 1, further comprising identifying the CTCs derived from gastric cancer (GC) if the CTCs express the markers comprising CD45⁻, CK-7/8⁺, CK-14/15/16/19⁺, EpCAM⁺, and HLA-A.B.C⁺.

11. The method of claim 10, further comprising administrating to the individual at least one treatment selected from the group consisting of: a Her2 tyrosine kinase inhibitors, an anti-Her2 antibody, a bispecific antibody, a Her2-targeting antibody drug conjugates, and Her2-targeted CAR-T cells if the CTCs is Her2⁺.

12. The method of claim 11, wherein the Her2 tyrosine kinase inhibitors comprises lapatinib; the anti-Her2 antibody comprises trastuzumab, pertuzumab, tucatinib and margetuximab; the bispecific antibody comprises zanidatamab; or the Her2-targeting antibody drug conjugates comprises trastuzumab emtansine and trastuzumab deruxtecan.

13. A method for treating a cancer in an individual in need thereof, comprising:
a) gating circulating tumor cells (CTCs) based on forward scatter (FSC), CD45, CK-7/8, CK-14/15/16/19, EpCAM, HLA-A.B.C, and VIM in a biological sample derived from the individual;
b) testing the CTCs for mutated epithelial growth factor receptor (mEGFR) L858R or Her2; and
c) administrating to the individual a cancer therapy to the individual, wherein
i) the cancer therapy is an EGFR tyrosine kinase inhibitor, if the cancer is identified as non-small cell lung cancer (NSCLC) with the CTCs is mEGFR L858R⁺, or
ii) the cancer therapy comprises at least one treatment selected from the group consisting of: a Her2 tyrosine kinase inhibitors, an anti-Her2 antibody, a bispecific antibody, a Her2-targeting antibody drug conjugates, and Her2-targeted CAR-T cells if the cancer is identified as gastric cancer (GC) with the CTCs is Her2⁺.

14. The method of claim 13, wherein the EGFR tyrosine kinase inhibitor comprises erlotinib, gefitinib, icotininib, afatinib, dacomitinib, osimertinib, rociletinib, olmutinib, lazertinib and zorifertinib.

15. The method of claim 13, wherein the Her2 tyrosine kinase inhibitors comprises lapatinib; the anti-Her2 antibody comprises trastuzumab, pertuzumab, tucatinib and margetuximab; the bispecific antibody comprises zanidatamab; or the Her2-targeting antibody drug conjugates comprises trastuzumab emtansine and trastuzumab deruxtecan.

16. A method for identifying an individual with a cancer likely to respond to treatment with at least one treatment selected from the group consisting of: an EGFR tyrosine kinase inhibitor, a Her2 tyrosine kinase inhibitors, an anti-Her2 antibody, a Her2-targeting antibody drug conjugates, and Her2-targeted CAR-T cells:
a) gating circulating tumor cells (CTCs) based on forward scatter (FSC), CD45, CK-7/8, CK-14/15/16/19, EpCAM, HLA-A.B.C, and VIM in a biological sample derived from the individual;
b) testing the CTCs for mutated epithelial growth factor receptor (mEGFR) L858R or Her2; and
c) identifying an individual with a cancer likely to respond to treatment with
i) an EGFR tyrosine kinase inhibitor, if the CTCs are derived from NSCLC and have CD45CK⁻7/8⁺CK-14/15/16/19⁻EpCAM⁺HLA-A.B.C⁺VIM⁺mEGFR L858R⁺; or
ii) at least one treatment selected from the group consisting of: a Her2 tyrosine kinase inhibitors, an anti-Her2 antibody, a bispecific antibody, a Her2-targeting antibody drug conjugates, and Her2-targeted CAR-T cells if the CTCs are derived from GC and have CD45⁻CK-7/8⁺CK-14/15/16/19⁺EpCAM⁺HLA-A.B.C⁺Her2⁺.

17. A kit for use in the method of claim 1, comprising one or more reagents for labeling CD45, CK-7/8, CK-14/15/16/19, EpCAM, HLA-A.B.C, VIM, mEGFR L858R and/or Her2.
